(19) Europäisches Patentamt
European Patent Office
Office européen des brevets

(11) Publication number: **0 478 292 A1**

(12) **EUROPEAN PATENT APPLICATION**

(21) Application number: **91308716.9**

(51) Int. Cl.[5]: **C07F 9/6561,** A61K 31/675

(22) Date of filing: **25.09.91**

(30) Priority: **26.09.90 GB 9020932**

(43) Date of publication of application:
**01.04.92 Bulletin 92/14**

(84) Designated Contracting States:
**AT BE CH DE DK ES FR GB GR IT LI LU NL SE**

(71) Applicant: **THE WELLCOME FOUNDATION LIMITED**
**Unicorn House 160 Euston Road**
**London NW1 2BP (GB)**

(72) Inventor: **Kelley, James Leroy**
**10928 Raven Rock Drive**
**Raleigh, North Carolina 27614 (US)**
Inventor: **McLean, Ed Williams**
**3208 Coleridge Drive**
**Raleigh, North Carolina 27609 (US)**

(74) Representative: **Rollins, Anthony John et al**
**Group Patents & Agreements The Wellcome Research Laboratories Langley Court**
**Beckenham Kent BR3 3BS (GB)**

(54) **Heterocyclic compounds.**

(57) Compounds of the formula (I):

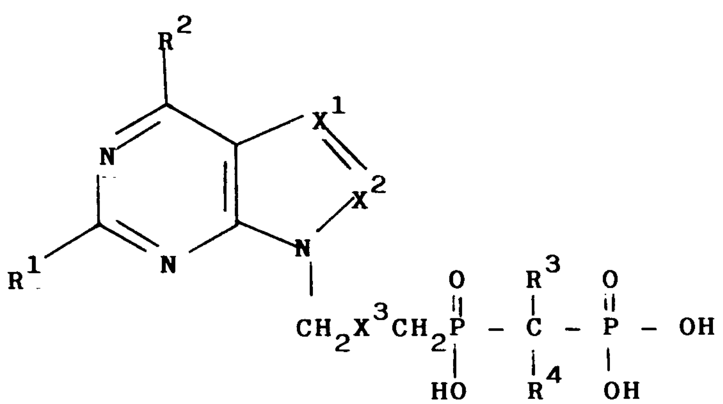

(I)

or a pharmaceutically acceptable salt or ester thereof, wherein $X^1$, is nitrogen or a group CH and $X^2$ is nitrogen or a group $CR^5$ wherein $R^5$ is hydrogen, hydroxy, halo or a group $NHR^6$ wherein $R^6$ is hydrogen or a $C_{1-4}$ alkyl or $C_{1-4}$ alkylcarbonyl group, $R^1$ is amino or hydrogen; $R^2$ is hydrogen, halo, SH, a group $NHR^7$ wherein $R^7$ is hydrogen or a $C_{1-4}$ alkyl or $C_{1-4}$ alkylcarbonyl group or $R^2$ is a group $OR^8$ wherein $R^8$ is hydrogen, $C_{1-4}$ alkyl or benzyl, $R^3$ and $R^4$ are the same or different and each is hydrogen or halo, $X^3$ is a chain containing m $CH_2$ groups and n oxygen atoms, m being 1, 2, 3, 4, or 5 and n being 0, 1 or 2 the sum of m and n being 2, 3, 4, 5, or 6; are disclosed as inhibitors of the enzyme, purine nucleoside phosphorylase, pharmaceutical compositions containing the compounds, their use in medicine and processes for their preparation are also described.

EP 0 478 292 A1

The present invention relates to nitrogen containing bicyclic aromatic ring systems, and particularly purines, substituted by a ((phosphinico)methyl)phosphonic acid residue which are inhibitors of purine nucleoside phosphorylase (PNP), to their preparation, to pharmaceutical compositions containing them and to their use in medicine

PNP is a purine-metabolising enzyme which under normal in-vivo conditions catalyses the reversible phosphorolysis of purine nucleosides such as (deoxy)inosine and (deoxy)guanosine to their respective bases, hypoxanthine and guanine and the corresponding (deoxy)ribose-1-phosphate.

It has been reported (J.Clin.Invest., 74,(3),951-5) that PNP deficiency in humans is associated with T-cell immuno-deficiency and that selective toxicity to T-cells rather than B-cells can be induced by administering a cytotoxic substrate of PNP (deoxyguanosine). It has been suggested that this selective toxicity would be of advantage in the treatment of T-cell disorders, i.e. T-cell leukemia, and autoimmune diseases, i.e. in the suppression of host-vs-graft response in organ transplant, rheumatoid arthritis, multiple sclerosis, systemic lupus erythematosus, type 1 diabetes and myasthemia gravis and the treatment of gout and other hyperuricemic states and psoriasis.

Acyclovir diphosphate has been reported (J.Biol.Chem., (1984), 259, 4065-9) to be a potent PNP inhibitor.

A series of guaninyl ((phosphinico)methyl)phosphonic acids have now been discovered which are potent PNP inhibitors.

Accordingly, the present invention provides a compound of the formula (I):

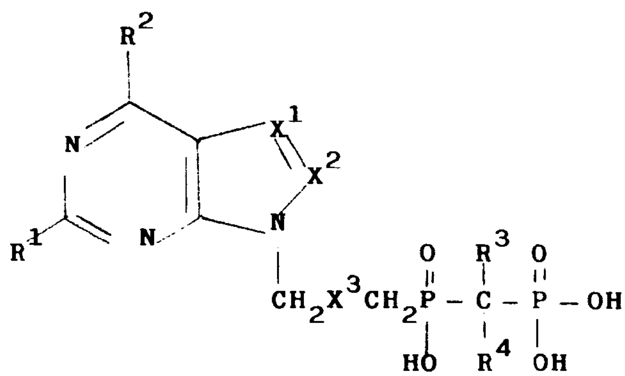

(I)

or a pharmaceutically acceptable salt or ester thereof, wherein $X^1$, is nitrogen or a group CH and $X^2$ is nitrogen or a group $CR^5$ wherein $R^5$ is hydrogen, hydroxy, halo or a group $NHR^6$ wherein $R^6$ is hydrogen or a $C_{1-4}$ alkyl or $C_{1-4}$ alkylcarbonyl group, $R^1$ is amino or hydrogen; $R^2$ is hydrogen, halo, SH, a group $NHR^7$ wherein $R^7$ is hydrogen or a $C_{1-4}$ alkyl or $C_{1-4}$ alkylcarbonyl group or $R^2$ is a group $OR^8$ wherein $R^8$ is hydrogen, $C_{1-4}$ alkyl or benzyl, $R^3$ and $R^4$ are the same or different and each is hydrogen or halo, $X^3$ is a chain containing m $CH_2$ groups and n oxygen atoms, m being 1, 2, 3, 4, or 5 and n being 0, 1 or 2 the sum of m and n being 2, 3, 4, 5, or 6.

Preferably $X^1$ is nitrogen. Suitably $X^2$ is nitrogen, or a group $CR^5$ wherein $R^5$ is hydrogen, halo or $NH_2$.

Suitably $R^1$ is hydrogen or $NH_2$.

Preferably $R^2$ is hydroxy.

Suitably $R^3$ and $R^4$ are each hydrogen or fluoro, preferably both are hydrogen.

Preferably n is 0 or 1.

Suitably the sum of m and n is 2, 3, 4 or 5 and preferably the sum of m and n is 3 or 4.

Preferably $X^3$ is a group $(CH_2)_3$ or $(CH_2)_4$ or $(CH_2)_2O$.

Preferred compounds of the present invention include:

(((4-(2-amino-1,6-dihydro-6-oxo-9H-purin-9-yl)butyl)phosphinico)methyl)phosphonic acid

(((5-(2-amino-1,6-dihydro-6-oxo-9H-purin-9-yl)pentyl)phosphinico)methyl)phosphonic acid

(((6-(2-amino-1,6-dihydro-6-oxo-9H-purin-9-yl)hexyl)phosphinico)methyl)phosphonic acid

(((7-(2-amino-1,6-dihydro-6-oxo-9H-purin-9-yl)heptyl)phosphonico)methyl)phosphonic acid.

(((3-(Amino-1,6-dihydro-6-oxo-9H-purine-9-yl)propoxy)methyl)phosphinico)methyl)phosphonic acid.

The compounds of the formula (I) may exist in tautomeric forms, the present invention relates to tautomers of the compounds of the formula (I) as well as the form of the compound of the formula (I) drawn.

Salts of the compounds of the present invention, may comprise acid addition salts derived from an amino group or anionic species derived from the hydroxy groups of formula (I), and a cation. In both types of salts, the therapeutic activity resides in the moiety derived from the compound of the invention as defined herein and

the identity of the other component is of less importance although for therapeutic and prophylactic purposes it is, preferably, pharmaceutically acceptable to the patient. Examples of pharmaceutically acceptable acid addition salts include those derived from mineral acids, such as hydrochloric, hydrobromic, phosphoric, metaphosphoric, nitric and sulphuric acids, and organic acids, such as tartaric, acetic, trifluoroacetic, citric, malic, lactic, fumaric, benzoic, glycollic, gluconic, succinic and methanesulphonic and arylsulphonic, for example p-toluenesulphonic, acids. Examples of salts comprising an anionic species derived from a compound of formula (I) and a cation include ammonium salts, alkali metal salts, such as sodium and potassium salts, alkaline earth salts, such as magnesium, zinc and calcium salts, and salts formed with organic bases, for example, amino salts derived from mono-, di- or tri-(lower alkyl) or (lower alkanol)amines, such as triethanolamine and diethylaminoethylamine, and salts with heterocyclic amines such as piperidine, pyridine, piperazine and morpholine. The pharmaceutically acceptable salts together with the salts which are not thus acceptable have utility in the isolation and/or the purification of the compounds of the invention, and the pharmaceutically unacceptable salts are also useful in being convertible to the pharmaceutically acceptable salts by techniques well known in the art.

Esters of the compounds of the present invention include $C_{1-6}$ branched or straight chain alkyl (e.g. methyl, ethyl, n-propyl, i-propyl, t-butyl, etc.), $C_{1-6}$ alkoxyalkyl (e.g. methoxymethyl), $C_{7-8}$ aralkyl (e.g. benzyl), or phenyl esters each optionally substituted by halo, $C_{1-4}$ alkyl, $C_{1-4}$ alkoxy, nitro or amino. As the compounds of the present invention are ((phosphinico)methyl)phosphonic acids, there may be one, two or three ester groups present. Esters of the compounds of the formula (I) are useful intermediates in the preparation of compounds of the formula (I) and have potential utility as prodrug forms.

The compounds of the present invention exhibit enzyme inhibitory activity against purine nucleoside phosphorylase (PNP). Since T-cells play a central role in immune response, use of the compounds of the invention is contemplated for the immunoregulation of autoimmune disease such as rheumatoid arthritis, systemic lupus erythematosus, inflammatory bowel disease, multiple sclerosis, myasthemia gravis, transplantation, juvenile diabetes, cancer and viral diseases. The present invention thus provides a method of treating disease such as autoimmune disease characterised by abnormal immune response in animals by the administration of an effective amount of a compound of the formula (I).

The compound of formula (I) and pharmaceutically acceptable derivatives thereof (hereafter collectively referred to as the active ingredients) may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with, for example, the condition of the recipient but, in general, oral and parenteral administration is preferred.

For each of the above-indicated utilities and indications the amount required of an active ingredient (as above defined) will depend upon a number of factors including the severity of the condition to be treated and the identity of the recipient and will ultimately be at the discretion of the attendant physician. In general, however, for each of these utilities and indications, a suitable, effective dose will be in the range 0.1 to 600 mg per kilogram body weight of recipient per day, most preferably in the range 7.0 to 200 mg per kilogram body weight per day; an optimum dose is about 50 mg per kilogram body weight per day (unless otherwise indicated all weights of active ingredient are calculated as the parent compound of formula (I); for salts and esters thereof the figures would be increased proportionately). The desired dose is preferably presented as two, three, four or more sub-doses administered at appropriate intervals throughout the day. These sub-doses may be administered in unit dosage forms, for example, containing 250 to 1500 mg, preferably 500 to 1000 mg of active ingredient per unit dosage form.

While it is possible for the active ingredients to be administered alone it is preferable to present them as pharmaceutical formulations. The formulations of the present invention comprise at least one active ingredient, as above defined, together with one or more acceptable carriers thereof and optionally other therapeutic ingredients. The carrier(s) must be "acceptable" in the sense of being compatible with the other ingredients of the formulation and not deleterious to the recipients thereof.

The formulations include those suitable for oral, rectal,nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units

such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compression tablets may be prepared by compressing in an suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide the desired release profile.

For infections of the eye or other external tissues, e.g., mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream may include, for example, at least 30% w/w of a polyhydric alcohol, i.e. an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and related analogues.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with an lipophilic emulsifier which acts as a stabilizer. It is also preferred to include both an oil and a fat. Together, the emulsifier(s) with or without stabilizer(s) make up the so-called emulsifying wax, and the wax together with the oil and/or fat make up the so-called emulsifying ointment base which forms the oily dispersed phase of the cream formulations.

Emulgents and emulsion stabilizers suitable for use in the formulation of the present invention include Tween 60, Span 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono-or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured base, usually sucrose and acacia or tragacanth; pastilles comprising the active ingredient in an inert base such as gelatin and glycerin, or sucrose and acacia; and mouth-washes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known

in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The present invention also provides a process for the preparation of a compound of formula I, or a pharmaceutically acceptable salt or ester thereof, comprising:

a) the reaction of a compound

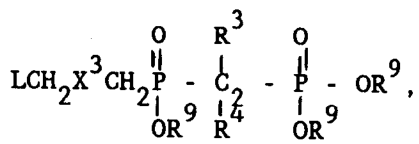

wherein $R^9$ is a $C_{1-6}$ alkyl or C7-8 aralkyl protecting group and L is a leaving group, with a compound of the formula (II):

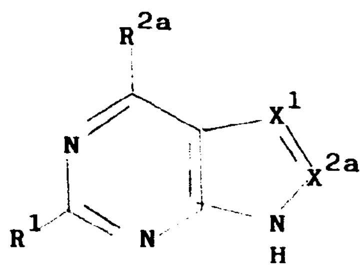

(II)

wherein $R^1$ and $X^1$ are as hereinbefore defined and $R^{2a}$ and $X^{2a}$ are groups $R^2$ and $X^2$ as hereinbefore defined or such groups substituted by a suitable protecting group, and thereafter optionally removing any protecting groups present

b) when $X^3$ contains one or two oxygen atoms, the reaction of a compound

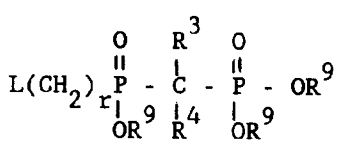

wherein L $R^3$, $R^4$ and $R^9$ are as hereinbefore defined, with a compound of the formula (III):

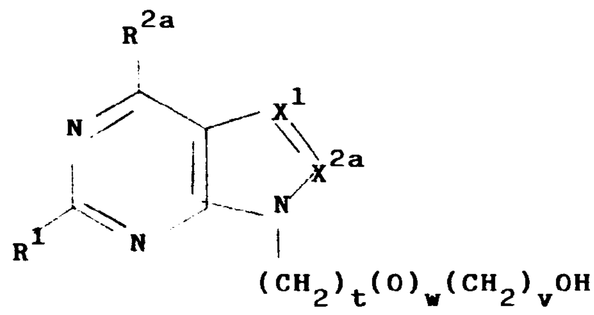

(III)

wherein $R^1$, $R^{2a}$, $X^1$ and $X^{2a}$ are as hereinbefore defined and t is 1, 2, 3, or 4, w is 0 or 1 and v is 0, 1, 2 or 3, the sum of r, t, w and v being 5, 6 or 7.

c) the conversion of one compound of the formula (I) from another compound of the formula (I), for example when $R^2$ is hydroxy, the hydrolysis of a compound of the formula (IV):

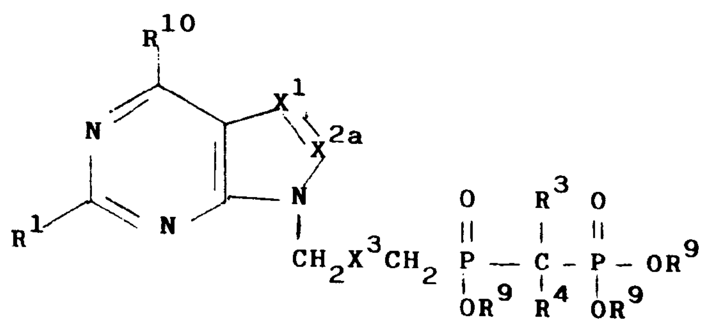

(IV)

wherein $R^1$, $X^1$, $X^{2a}$, $X^3$, $R^3$, $R^4$ and $R^9$ are as hereinbefore defined and $R^{10}$ is halo.

Process (a) is suitably carried out at a non-extreme temperature, i.e. between 0° and 120°C and conveniently between 50° and 90°C, in a dipolar aprotic solvent such as dimethylformamide in the presence of a base, such as cesium carbonate. The leaving group is conveniently a halo atom or a substituted sulphonyloxy group, for example a methanesulphonyloxy group. When $R^2$ is a hydroxy or thiol group and $R^5$ is a hydroxy group, these are suitably protected by an alkoxylalkyl group, for example a methoxyethyl group.

The protecting groups are conveniently removed by acid hydrolysis, for example using hydrochloric acid, at a non-extreme temperature, for example between 20°C and 100°C.

Process (b) is conveniently carried out at a non-extreme temperature, i.e. between 0° and 120°C, in a dipolar aprotic solvent, such as dimethylformamide, optionally in the presence of a strong base. Suitable leaving groups are as described for process (a) above.

Process (c), the conversion of a group $R^{10}$ and $OR^9$ to a hydroxy group is suitably carried out in the presence of acid, for example concentrated hydrochloric acid at a non-extreme temperature, i.e. between 0° and 120°C, and suitably between 70°C and 100°C.

The intermediates of the formula (III) and (IV) may conveniently be prepared in analogous manner to process (a).

The following examples serve to illustrate the present invention.

Example 1

Preparation of (((5-(2-amino-1,6-dihydro-6-oxo-9H-purin-9-yl)pentyl) phosphinico)methyl)phosphonic acid (compound 1)

A) Preparation of ethyl hydrogen 4-pentenylphosphonate

Diethyl 4-pentenylphosphonate (26.0g, 0.126moles) (D.Loewus, J.Am.Chem.Soc., (1981), 103, 2292) and 85% aqueous potassium hydroxide (33.3g, 0.504moles) were combined in ethanol (250ml) and refluxed under a nitrogen atmosphere for 1.5 hours. The reaction solution was spin evaporated *in vacuo* to one half volume, diluted to 800ml with distilled water, and cooled in an ice bath. The solution was made acidic with concentrated hydrochloric acid (12N) (50ml) and extracted with dichloromethane (3 x 250ml). The dichloromethane extracts

were concentrated by spin evaporation in vacuo to give 23.75g (106%) of ethyl hydrogen 4-pentenylphosphonate as a clear oil that was contaminated with a small amount of residual water: $^1H$ nmr ($CDCl_3$): δ 9.66 (s, 1H, OH), 5.8-5.6 (m, 2H, $CH_2$=C), 5.05-4.92 (m, 1H, CH), 4.15-3.95 (m, 2H, $OCH_2$), 2.15-2.05 (m, 2H, $CH_2$), 1.80-1.62 (m, 4H, $PCH_2CH_2$), 1.31 (t, 3H, J = 6.9 Hz, $CH_3$).

B) Preparation of ethyl 4-pentenylphosphonochloridate

To a stirred solution of ethyl hydrogen 4-pentenylphosphonate (5.0g, 0.028moles) in dichloromethane (50ml) at 0°C was added dimethylformamide (0.5ml) and oxalyl chloride (6.4g, 0.0505moles). The reaction was allowed to come to room temperature without external heat and was stirred for 3 days while protected from moisture by a calcium chloride drying tube. The volatiles were removed by spin evaporation in vacuo at <40°C with the addition of dichloromethane to aid in codistillation to give 5.55g (100%) of ethyl 4-pentenylphosphonochloridate which was used without further purification: $^1H$ nmr ($CDCl_3$): δ 5.85-5.66 (m, 2H, $CH_2$=C), 5.10-4.95 (m, 1H, CH), 4.44-4.05 (m, 2H, $OCH_2$), 2.25-2.03 (m, 4H, $PCH_2$ and $CH_2C$=C), 1.90-1.62 (m, 2H, $CH_2$), 1.40-1.25 (t, 3H, J = 7.3 Hz, $CH_3$).

C) Préparation of diethyl ((ethoxy(4-pentenyl)phosphinoyl)methyl)phosphonate

A solution of n-butyl lithium (1.6M in hexane) (87.5ml, 0.140moles), was addred dropwise with stirring to diethyl methylphosphonate (21.3g, 0.140moles) (Aldrich Chemical Company) in 200ml of anhydrous tetrahydrofuran (3Å sieve dried) at -60°C under a nitrogen atmosphere. After 10 minutes, ethyl 4-pentenylphosphonochloridate (11.0g, 0.056moles) in tetrahydrofuran (40ml) was added slowly and stirring was continued. After 2.5 hours at -60°C, the excess base was neutralized with aqueous ammonium chloride. The reaction solution was diluted with water (600ml) and extracted with dichloromethane (3 x 200ml). The dichloromethane layers were combined and spin evaporated in vacuo. The residue was distilled to give 9.3g (53%) of diethyl ((ethoxy(4-pentenyl)phosphinoyl)methyl)phosphonate: bp 155-165°C at 0.1 Torr: $^1H$ nmr ($CDCl_3$): δ 5.80-5.65 (m, 2H, $CH_2$=C), 5.10-4.93 (m, 1H, CH), 4.20-4.00 (m, 6H, $CH_2$), 2.36 (dd, 2H, J = 16.5 Hz, 20.8, $PCH_2P$), 2.14 (broad q, 2H, J = 7.0 Hz, C=$CHCH_2$), 2.0-1.84 (m, 2H, $PCH_2$), 1.80-1.65 (m, 2H, $CH_2$), 1.32 (t, 6H, J = 7.1 Hz, $CH_3$), 1.309 (t, 3H, J = 7.1 Hz, $CH_3$).

D) Preparation of diethyl ((ethoxy(5-hydroxypentyl)phosphinoyl)methyl)phosphonate

Diethyl ((ethoxy(4-pentenyl)phosphinoyl)methyl)phosphonate (5.0g, 0.016moles) in anhydrous tetrahydrofuran (15ml) was slowly added to 1.0M borane in tetrahydrofuran (16ml, 16mmoles) in anhydrous tetrahydrofuran (10ml) at -10°C under a nitrogen atmosphere. The solution was allowed to warm to room temperature (22°C) over 2 hours and was then cooled to 10°C. Very slowly, in a dropwise manner to control frothing, 3N aqueous sodium hydroxide (5.25ml) was added, followed by 30% hydrogen peroxide (5.25ml), while maintaining the temperature by ice bath cooling. The reaction was then heated to 50°C for 1.5 hours and cooled to 25°C. The excess peroxide was reduced with aqueous sodium bisulfite (5%). The volatiles were then removed by spin evaporation in vacuo. Dichloromethane was added during the evaporation to assist in removal of the residual water as its azeotrope. The damp solid residue was extracted with ethyl acetate (3 x 150ml) which was then dried with magnesium sulfate, filtered, and concentrated in vacuo to give 4.68g (88.6%) of diethyl ((ethoxy(5-hydroxypenty)phosphinoyl)methyl)phosphonate as a clear oil: $^1H$ nmr ($CDCl_3$): δ 4.25-4.00 (m, 6H, $OCH_2$), 3.64 (t, 2H, J = 6.0 Hz, $HOCH_2$), 2.38 (dd, 2H, $J_{PH}$=16.6 and 20.7 Hz, $PCH_2P$), 2.05-1.85 (m, 2H, $PCH_2$), 1.75-1.40 (m, 6H, $CH_2CH_2CH_2$), 1.337 (t, 6H, J = 7.0 Hz, $CH_3$), 1.326 (t, 3H, J = 7.0 Hz, $CH_3$).

E) Preparation of diethyl ((ethoxy(5-((methylsulfonyl)oxy)pentyl)phosphinoyl)methyl)phosphonate

Methanesulfonyl chloride (3.04ml), 39.4mmoles) was slowly added to a stirred solution of diethyl ((ethoxy(5-hydroxypentyl)phosphinoyl)methyl)phosphonate (13.0g, 39.4mmoles) and triethylamine (5.5ml, 39.4mmoles) in dichloromethane (50ml) at -60°C. After stirring for 3 hours, the reaction mixture was applied directly to a chromatography column (5cm x 20cm) of Silica Gel 60 wet with dichloromethane and was purified by chromatographic elution (eluent: methanol gradient in dichloromethane, 0% to 10%, 3 1). Evaporation of the appropriate fractions at <40°C gave 8.75g (54.4%) of diethyl ((ethoxy(5-((methylsulfonyl)oxy)pentyl)phosphinoyl)methyl)phosphonate as a clear oil: ms CI ($CH_4$): m/e = 409 ($MH^+$); $^1H$ nmr ($CDCl_3$) δ 4.25-4.00 (m, 8H, $OCH_2$), 2.99 (s, 3H, $SCH_3$), 2.36 (dd, 2H, $J_{PH}$ = 16.6 and 20.7 Hz, $PCH_2P$), 2.05-1.4 (m, 8H, $PCH_2CH_2CH_2CH_2$), 1.33 (t, 3H, J = 7.03 Hz, $CH_3$), 1.31 (t, 3H, J = 7.03 Hz, $CH_3$).

F) Preparation of diethyl (((5-(2-amino-6-(2-methoxyethoxy)-9H-purin-9-yl)pentyl)ethoxyphosphinoyl)methyl)phosphonate

Diethyl ((ethoxy(5-((methylsulfonyl)oxy)pentyl)phosphinoyl)methyl)phosphonate (33.45mmoles, 13.66g), 2-amino-6-(2-methoxyethoxy)-9H-purine (33.45mmol, 7.0g) (J. Kjellberg, M. Liljenberg, and N.G. Johansson, Tetrahedron Lett. 1986, 27, 877), and anhydrous cesium carbonate (66.9mmol, 21.7g) (Aldrich) in 200ml of anhydrous dimethylformamide (freshly distilled from calcium hydride) were stirred at 80°C for 3 hours under a nitrogen atmosphere. The dimethylformamide was evaporated in vacuo. The residue was dissolved in dichloromethane, filtered, and purified by flash chromatography on a column (5cm x 25cm) of Silica Gel 60 wet with dichloromethane (eluent: methanol gradient in dichloromethane, 0% to 10%, 4 1). Evaporation of the appropriate fractions at <40°C gave 6.04g (34.4%) of diethyl (((5-(2-amino-6-(2-methoxyethoxy)-9H-purin-9-yl)pentyl)ethoxyphosphinoyl)methyl)phosphonate as a pale oil: $^1H$ nmr ($CDCl_3$): δ 7.57 (s, 1H, H-8), 4.94 (broad s, 2H, $NH_2$), 4.63 (t, 2H, J = 5.1 Hz, $OCH_2$), 4.25-4.00 (m, 8H, $NCH_2$ and $POCH_2$); 3.80 (t, 2H, J = 5.1 Hz, $OCH_2$), 3.42 (s, 3H, $OCH_3$), 2.37 (dd, 2H, $J_{PH}$ = 16.6 and 20.7 Hz, $PCH_2P$), 2.00-1.60 (m, 6H, $PCH_2$ and $CH_2CH_2$), 1.48-1.324 (m, 2H, $CH_2$), 1.32 (t, 3H, J = 7.03 Hz, $CH_3$), 1.31 (t, 3H, J = 7.03 Hz, $CH_3$).

G) Preparation of (((5-(2-amino-1,6-dihydro-6-oxo-9H-purin-9-yl)pentyl)phosphinico)methyl)phosphonic acid

Diethyl (((5-(2-amino-6-(2-methoxyethoxy)-9H-purin-9-yl)pentyl)ethoxyphosphinoyl)methyl)phosphonate (4.0g, 0.0567moles) was hydrolyzed by heating in hydrochloric acid (12M) (40ml) at 100°C for 18 hours. The solution was concentrated by spin evaporation in vacuo. The residue was dissolved in water and purified by anion exchange chromatography on DEAE Sephadex A-25 ($-HCO_3$ form) with an aqueous ammonium bicarbonate buffer gradient (0 to 1M). The appropriate fractions were combined, spin evaporated in vacuo to a small volume, and lyopholized to give 2.09g (71%) of (((5-(2-amino-1,6-dihydro-6-oxo-9H-purin-9-yl)pentyl)phosphinico)methyl)phosphonic acid as a white powder: mp=152-155°C; ms FAB+: m/e = 380 ($MH^+$); uv (0.1N hydrochloric acid): λmax 253 (ε 10900), 278 (ε 7300), λmin 227 (2700), 270 (ε 7000); (pH7 buffer): λmax 271 (ε 8400), 252 (ε 10900); λmin 228 (ε 3800), 267 (ε 8300); (0.1N sodium hydroxide): 270 (ε 9500), λmin 233 (ε 4600), λsh 257 (ε 8700); $^1H$ nmr (DMSO.$d_6$): δ 7.69 (s, 1H, H-8), 6.6 (br s, 2H, $NH_2$), 4.5 (very broad s, $+NH_4$ and $H_2O$), 3.91 (t, 2H, J = 6.8 Hz, $NCH_2$), 1.80-1.60 (m, 4H, $PCH_2P$ and $CH_2$), 1.58-1.40 (m, 4H, $PCH_2$ and $CH_2$), 1.38-1.20 (m, 2H, $CH_2$); $^{31}P$ nmr (DMSO-$d_6$): δ 35.205 (s, 1P, phosphinyl), 14.484 (s, 1P, phosphonyl).

Example 2

Preparation of (((4-(2-amino-1,6-dihydro-6-oxo-9H-purin-9-yl)butyl)phosphinico)methyl)phosphonic acid (compound 2)

A) Preparation of ethyl 3-butenylphosphonochloridate

Phosphorous pentachloride (6.9g, 30.2mmoles) was added in one portion to a solution of diethyl 3-butenylphosphonate (4.8g, 25.2mmoles) (J.Kabak, L.DeFelippe, R.Engel, and B.Tropp, J.Med.Chem., (1972), 15, 1074) in dichloromethane (40ml). The reaction was stoppered and stirred at room temperature for 4 hours. Fractional distillation gave 2.43g (53%) of ethyl 3-butenylphosphonochloridate; bp 123-125°C at 15 Torr; $^1H$ nmr ($CDCl_3$): δ 5.85-5.70 (m, 1H, $CH_2=C\underline{H}$), 5.15-4.95 (m, 2H, $C\underline{H_2}=CH$), 4.35-4.10 (m, 2H, $POCH_2$), 2.48-2.35 (m, 2H, $C_2=CHC\underline{H_2}$), 2.25-2.10 (m, 2H, $PCH_2$), 1.35 (t, 3H, J = 7.1 Hz, $POCH_2C\underline{H_3}$).

B) Preparation of diethyl (((3-butenyl)ethoxyphosphinoyl)methyl)phosphonate

This compound was prepared in an analogous manner to that of Example 1 with the replacement of ethyl 4-pentenylphosphonochloridate) in Example 1C with ethyl 3-butenylphosphonochloridate. Fractional distillation gave 4.42g (21%) of diethyl (((3-butenyl)ethoxyphosphinoyl)methyl)phosphonate hemihydrate; bp 166-167°C at 0.15 Torr; ms CI ($CH_4$): m/e = 299 ($MH^+$); $^1H$ nmr ($CDCl_3$): δ 5.90-5.70 (m, 1H, CH), 5.10-4.93 (m, 2H, $CH_2=C$), 4.20-3.95 (m, 6H, $OCH_2$), 2.35 (dd, 2H, $J_{PH}$=16.5, 20.8 Hz, $PCH_2P$), 2.45-2.20 (m, 2H, $CH_2$), 2.10-1.90 (m, 2H, $PCH_2$), 1.29 (t, 3H, J = 8.0, $CH_3$); $^{31}P$ nmr ($CDCl_3$): δ 47.14 (d, 1P, $J_{PH}$ = 5.4 Hz, phosphinyl), 22.25 (d, 1P, $J_{PH}$ = 5.4 Hz, phosphonyl).

C) Preparation of diethyl ((ethoxy(4-hydroxybutyl)phosphinoyl)methyl)phosphonate

This compound was prepared in an analogous manner to that of Example 1 with the replacement of diethyl

((ethoxy(4-pentenyl)phosphinoyl)methyl)phosphonate in Example 1D with diethyl (((3-butenyl)ethoxyphosphinoyl)methyl)phosphonate. The residue obtained from concentration of the ethyl acetate extracts was dissolved in dichloromethane (100ml), filtered and spin evaporated in vacuo, with the addition of dichloromethane (2 x 100ml), to give 11.64g (81.6%) of diethyl ((ethoxy(4-hydroxybutyl)phosphinoyl)methyl)-phosphonate 1.2 hydrate as a clear oil: ms CI ($CH_4$): m/e=317 ($MH^+$); $^1H$ nmr (DMSO.$d_6$): δ 3.90-4.10 (m, 6H, $POC\underline{H}_2CH_3$), 3.39 (t, 2H, J = 5.9 Hz $C\underline{H}_2OH$), 2.62 (dd, 1H, $J_{PH}$ = 16.8 and 20.2 Hz, $PC\underline{H}HP$), 2.61 (dd, 1H, $J_{PH}$ = 16.4 and 20.4 Hz, $PCH\underline{H}P$), 1.90-1.76 (m, 2H, $PCH_2$), 1.58-1.42 (m, 4H, $PCH_2C\underline{H}_2C\underline{H}_2$), 1.24 (t, 6H, J = 7.0 Hz, $POCH_2C\underline{H}_3$), 1.22 (t, 3H, J = 7.0 Hz, $POCH_2C\underline{H}_3$); $^{31}P$ nmr (DMSO.$d_6$): δ 48.08 (d, 1P. $J_{PH}$ = 5.3 Hz, phosphinyl), 22.66 (d, 1P, J = 5.3 Hz, phosphonyl).

D) Preparation of diethyl ((ethoxy(4-((methylsulfonyl)oxy)butyl)phosphinoyl)methyl)phosphonate

This compound was prepared in an analogous manner to that of Example 1 with the replacement of diethyl ((ethoxy(5-hydroxypentyl)phosphinoyl)methyl)phosphonate in Example 1E with diethyl ((ethoxy(4-hydroxybutyl)phosphinoyl)methyl)phosphonate. The chromatography solutions were spin evaporated in vacuo to give 3.43g (58%) of diethyl ((ethoxy(4-((methylsulfonyl)oxy)butyl)phosphinoyl)methyl)phosphonate as a clear oil: $^1H$ nmr ($CDCl_3$): δ 4.23 (t, 2H, J = 6.1 Hz, $SOCH_2$), 4.20-4.06 (m, 6H, $OCH_2$), 3.00 (s, 3H, $CH_3S$), 2.37 (dd, 2H, $J_{PH}$ = 16.7 and 20.6 Hz, $PCH_2P$), 2.05-1.70 (m, 6H, $CH_2CH_2CH_2$), 1.33 (t, 6H, J = 6.3 Hz, $CH_3$), 1.32 (t, 3H, $CH_3$), $^{31}P$ NMR (DMSO-$d_6$): δ 47.77 (d, 1P, J = 5.4 Hz, phosphinyl), 22.56 (d, 1P, J = 5.4 Hz, phosphonyl).

E) Preparation of diethyl (((4-(2-amino-6-(2-methoxyethoxy)-9H-purin-9-yl)butyl)ethoxyphosphinoyl)methyl)phosphonate

This compound was prepared in an analogous manner to that of Example 1 with the replacement of diethyl ((ethoxy(5-((methylsulfonyl)oxy)pentyl)phosphinoyl)methyl)phosphonate in Example 1F with diethyl ((ethoxy(4-((methylsulfonyl)oxy)butyl)phosphinoyl)methyl)phosphonate. The chromatography solution was concentrated in vacuo to give 1.52g (37.7%) of diethyl (((4-(2-amino-6-(2-methoxyethoxy-9H-purin-9-yl)butyl)ethoxyphosphinoyl)methyl)phosphonate as a clear oil: ms CI ($CH_4$): m/e = 508 ($MH^+$); $^1H$ nmr ($CDCl_3$): δ 7.59 (s, 1H, purine H-8), 4.91 (broad s, 2H, $NH_2$), 4.64 (t, 2H, J = 5.0 Hz, $OCH_2$), 4.20-4.05 (m, 6H, $OCH_2$), 4.07 (t, 2H, J = 7.2 Hz, $NCH_2$), 3.80 (t, 2H, J = 5.0 Hz, $OCH_2$), 3.43 (s, 3H, $CH_3$), 2.38 (dd, 2H, $J_{PH}$ = 16.7 and 20.7 Hz, $PCH_2P$), 2.12-1.90 (m, 4H, $CH_2CCH_2$), 1.75-1.55 (m, 2H, $CH_2$), 1.34 (t, 6H, J = 7.0 Hz, $CH_3$), 1.30 (t, 3H, J = 7.0 Hz, $CH_3$); $^{31}P$ nmr (DMSO.$d_6$): δ 47.75 (d, 1P, $J_{PH}$ - 5.15 Hz, phosphinyl), 22.57 (d, 1P, $J_{PH}$ = 5.15 Hz, phosphonyl).

F) Preparation of (((4-(2-amino-1.6-dihydro-6-oxo-9H-purin-9-yl)butyl)phosphinico)methyl)phosphonic acid

This compound was prepared in an analogous manner to that of Example 1 with the replacement of diethyl (((5-(2-amino-6-(2-methoxyethoxy)-9H-purin-9-yl)pentyl)ethoxyphosphinoyl)methyl)phosphonate in Example 1G with diethyl (((4-(2-amino-6-(2-methoxyethoxy)-9H-purin-9-yl)butyl)ethoxyphosphinoyl)methyl)phosphonate. Lyophilization of the chromatography solution gave 0.348g (29%) of (((4-(2-amino-1,6-dihydro-6-oxo-9H-purin-9-yl)butyl)phosphinico)methyl)phosphonic acid 1.7 ammonium salt 1.7 hydrate; mp = 173-176°C; ms ($FAB^+$): m/e = 366 ($MH^+$); $^1H$ nmr ($D_2O$) (HOD resonance was reduced by presaturation with the decoupler channel): δ 7.91 (broad s, 1H, purine 8-H), 4.08 (t, 2H, J = 7.0Hz, $NCH_2$), 2.00 (dd, 2H, $J_{PH}$ = 17 and 18 Hz, $PCH_2P$), 1.86 (quintet, 2H, J = 7.1 Hz, $NCH_2C\underline{H}_2$), 1.72 (broad s, 2H, $OCH_2$), 1.51 (broad s, 2H $PCH_2C\underline{H}_2$); $^{31}P$ nmr ($D_2O$) δ 39.16 (broad s, 1P, phosphinyl): uv (0.1N hydrochloric acid): λmax 254 (ε 11600), 279 (ε 7800); λmin 270 (ε 7400); (pH 7 buffer) λmax 254 (ε 12300), λsh 271 (ε 9300); (0.1N sodium hydroxide) λmax 270 (ε 10,500), λsh 258 (ε 9200).

Example 3

Preparation of (((6-(2-amino-1,6-dihydro-6-oxo-9H-purin-9-yl)hexyl)phosphinico)methyl)phosphonic acid 87.5% and 12.5% of the 7-purine isomer (compound 3)

A) Preparation of diethyl 5-hexenylphosphonate

A solution of 6-bromohexane (20.0g, 0.123moles) (Aldrich Chem.Co) and triethylphosphite (100ml) was heated in an oil bath at 170°C for 24 hours. The low boiling volatiles were collected in a Dean-Stark trap and discarded. Fractional distillation gave 21.25g (78.4%) of diethyl 5-hexenylphosphonate as a clear oil; bp 89-90°C at 0.1 Torr; ms CI ($CH_4$): m/e = 221 (MH+); $^1$H nmr ($CDCl_3$): δ 5.87-5.62 (m, 1H, $CH_2$-C$\underline{H}$), 5.05-4.88 (m, 2H, C$\underline{H_2}$=CH), 4.15-3.95 (m, 4H, POC$\underline{H_2}$), 2.04 (dt, 2H, J = 7.0 and 6.8 Hz, $CH_2$=CHC$\underline{H_2}$), 1.54-1.34 (m, 2H, $PCH_2$C$\underline{H_2}$), 1.29 (t, 6H, J = 7.0 Hz $POCH_2CH_3$).

B) Preparation of ethyl hydrogen 5-hexenylphosphonate

This compound was prepared in an analogous manner to that of Example 1 with the replacement of diethyl 4-pentenylphosphonate in Example 1A with diethyl 5-hexenylphosphonate. The dichloromethane extracts were concentrated in vacuo to give 12.91g (86.6%) of ethyl hydrogen 5-hexenylphosphonate as a clear oil: ms ($FAB^+$): m/e = 193 ($MH^+$; $^1$H nmr ($CDCl_3$): δ 11.95 (s, 1H, OH), 5.84-5.70 (m, 1H, CH), 5.04-4.90 (m, 2H, C$\underline{H_2}$-CH), 4.07 (dq, 2H, $J_{HH}$ = 7.12 Hz, $J_{PH}$ - 7.88 Hz, POC$\underline{H_2}$$CH_3$), 2.05 (dt, 2H, J = 6.8 Hz and 7.2 Hz, $CH_2$=CHC$\underline{H_2}$), 1.81-1.72 (m, 2H, PC$\underline{H_2}$), 1.71-1.55 (m, 2H, $PCH_2CH_2$C$\underline{H_2}$), 1.55-1.43 (m, 2H, $PCH_2$C$\underline{H_2}$), 1.31 (t, 3H, J = 7.0 Hz), $POCH_2CH_3$).

C) Preparation of ethyl 5-hexenylphosphonochloridate

This compound was prepared in an analogous manner to that of Example 1 with the replacement of ethyl hydrogen 4-pentenylphosphonate in Example 1B with ethyl hydrogen 5-hexenylphosphonate. Evaporation of the dichloromethane under reduced pressure left 12.7g (100%) of ethyl 5-hexenylphosphonochloridate as a clear oil contaminated with N,N-dimethylformamide (0.21 molar equivalents): $^1$H nmr ($CDCl_3$): δ 8.09 (broad s, 0.21 H, C$\underline{H}$O) 5.9-5.7 (m, 1H, $CH_2$=C$\underline{H}$); 5.1-4.9 (m, 2H, C$\underline{H_2}$=CH), 4.4-4.1 (m, 2H, POC$\underline{H_2}$$CH_3$), 3.03 (s, 0.63 H, NC$\underline{H_3}$), 2.94 (s, 0.63 H, NC$\underline{H_3}$), 2.2-2.0 (m, 4H, PC$\underline{H_2}$ and $CH_2$=CHC$\underline{H_2}$), 1.8-1.6 (m, 2H, $PCH_2CH_2$C$\underline{H_2}$), 1.60-1.45 (m, 2H, $PCH_2$C$\underline{H_2}$), 1.38 (t, 3H, J = 7.1 Hz, $POCH_2$C$\underline{H_3}$).

D) Preparation of diethyl ((ethoxy(5-hexenyl)phosphinoyl)methyl)phosphonate

This compound was prepared in an analogous manner to that of Example 1 with replacement of ethyl 4-pentenylphosphonochloridate in Example 1C with ethyl 5-hexenylphosphonochloridate. Fractional distillation gave 5.7g (30.7%) of diethyl ((ethoxy(5-hexenyl)phosphinoyl)methyl)phosphonate: bp = 160-180°C at 0.07 Torr: ms CI ($CH_4$): m/e = 327 ($MH^+$); $^1$H nmr ($CDCl_3$): δ 5.80-5.62 (m, 1H, $CH_2$=C$\underline{H}$), 5.00-4.88 (m, 2H, C$\underline{H_2}$=CH), 4.20-4.00 (m, 6H, POC$\underline{H_2}$), 3.51 (dd, 2H, $J_{PH}$ = 16.5 Hz and 20.7 Hz, $PCH_2P$), 2.08-1.99 (m, 2H, $CH_2$=CHC$\underline{H_2}$), 1.98-1.85 (m, 2H, PC$\underline{H_2}$), 1.68-1.53 (m, 2H, $CH_2$=$CHCH_2$C$\underline{H_2}$), 1.51-1.40 (m, 2H, $PCH_2$C$\underline{H_2}$), 1.31 (t, 4H, J = 7.0 Hz, $POCH_2CH_3$), 1.29 (t, 3H, J = 7.0 Hz, POC$\underline{H_2}$$CH_3$).

E) Preparation of diethyl ((ethoxy(6-hydroxyhexyl)phosphinoyl)methyl)phosphonate

This compound was prepared in an analogous manner to that of Example 1 with the replacement of diethyl ((ethoxy(4-pentenyl)phosphinoyl)methyl)phosphonate in Example 1D with diethyl ((ethoxy-(5-hexenyl)phosphinoyl)methyl)phosphonate. The ethyl acetate extraction solutions were concentrated in vacuo. The residue was dissolved in dichloromethane and chromatographed through a pad of Silica Gel 60 (3cm x 3cm) using a mixture of methanol and dichloromethane (1:20). The eluent solution was concentrated in vacuo with the addition of dichloromethane to give 4.96g (85.2%) of diethyl ((ethoxy(6-hydroxyhexyl)phosphinoyl)methyl)phosphonate monohydrate as a clear oil: ms CI($CH_4$): m/e = 345 ($MH^+$); $^1$H ($CDCl_3$): δ 4.22-4.01 (m, 6H, POC$\underline{H_2}$), 3.63 (t, 2H, J = 6.4 Hz, C$\underline{H_2}$OH), 2.38 (dd, 2H, J = 16.4 and 20.7 Hz, $PCH_2P$), 2.00-1.88 (m, 4H, C$\underline{H_2}$$CH_2$OH and PC$\underline{H_3}$), 1.7-1.6 (m, 2H, $PCH_2$C$\underline{H_2}$), 1.6-1.5 (m, 2H, $HOCH_2CH_2$C$\underline{H_2}$), 1.45-1.37 (m, 2H, $PCH_2CH_2$C$\underline{H_2}$), 1.34 (t, 6H, J = 7.0 Hz, $POCH_2$C$\underline{H_3}$), 1.33 (t, 3H, J = 7.0 Hz, $POCH_2CH_3$).

F) Preparation of diethyl ((ethoxy(6-((methylsulfonyl)oxy)hexyl)phosphinoyl)methyl)phosphonate

This compound was prepared in an analogous manner to that of Example 1 with the replacement of diethyl ((ethoxy(5-hydroxypentyl)phosphinoyl)methyl)phosphonate in Example 1E with diethyl ((ethoxy(6-hydroxyhexyl)phosphinoyl)methyl)phosphonate. Evaporation of the chromatography solutions in vacuo gave 2.48g (100%) of diethyl ((ethoxy(6-((methylsulfonyl)oxy)hexyl)phosphinoyl)methyl)phosphonate as a clear oil: $^1$H nmr ($CDCl_3$): δ 4.22 (t, 2H, J = 6.5 Hz, SOC$\underline{H_2}$), 4.25-4.15 (m, 6H, POC$\underline{H_2}$), 3.00 (s, 3H, $CH_3S$), 2.38 (dd, 2H, $J_{PH}$ = 16.4 and 20.7 Hz, $PCH_2P$), 2.0-1.89 (m, 2H, $PCH_2$), 1.80-1.70 (m, 2H, $SO_3CH_2$C$\underline{H_2}$), 1.70-1.60 (broad m, 2H, $PCH_2$C$\underline{H_2}$), 1.5-1.4 (m, 4H, $PCH_2CH_2$C$\underline{H_2}$C$\underline{H_2}$), 1.35 (t, 6H, J = 7.0 Hz, $POCH_2$C$\underline{H_3}$), 1.34 (t, 3H, J = 7.0 Hz, $POCH_3$C$\underline{H_3}$).

G) Preparation of (((6-(2-amino-1.6-dihydro-6-oxo-9H-purin-9-yl)hexyl)phosphinico)methyl)phosphonic acid 87,5% and 12.5% of the 7-purine isomer

To a mixture of 2-amino-6-chloropurine (3.11g, 18.4mmoles) (Sigma Chemical Co.) and potassium carbonate (5.0g, 36mmoles) in N,N-dimethylformamide (15ml), which had been dried by distillation from calcium hydride under nitrogen, was added a solution of diethyl ((ethoxy(6-((methylsulfonyl)oxo)hexyl)phosphinoyl) methyl)phosphonate (2.54g, 6.1mmoles) in N,N-dimethylformamide (5ml). The reaction mixture was heated to 80°C for 18 hours while protected form moisture. The solvent was removed by spin evaporation in vacuo and the residue was dissolved in aqueous hydrochloric acid (12N) (25ml) and refluxed for 24 hours. The solution was concentrated by spin evaporation in vacuo, distilled water (50ml) was added and removed by spin evaporation to remove residual hydrochloric acid. The residue was dissolved in water (25ml) and the pH was adjusted to 7 with potassium carbonate. After cooling in an ice bath for 5 hours, the precipitate was removed by filtration and the filtrates were diluted to 0.5 litres. Ion exchange column chromatography on QAE Sephadex A-25 ion exchange media (Pharmacia LKB Biotechnology Inc.) (-$HCO_3$ form, 2.5cm x 55cm column; 0.02-1.0M sodium carbonate buffer (pH 9.9), 2 1) was used for preliminary purification. Final purification was performed on DEAE Sephadex A-25 ion exchange media (Pharmacia LKB Biotechnology Inc.) (-$HCO_3$ form, 2.5cm x 55cm column, 0-1M ammonium bicarbonate buffer, 2 1). The chromatography solution was lyophilized to give 0.615g (23.7%) of (((6-(2-amino-1,6-dihydro-6-oxo-9H-purin-9-yl)hexyl)phosphinico)methyl)phosphonic acid 87.5% and 12.5% of the 7-purine isomer: mp = 173-175°C; ms $FAB^-$: m/e = 392 ($(M-H)^-$); uv (0.1N hydrochloric acid): λmax 278 (ε 8200); 253 (ε 12200), λmin 227 (ε 3300), 270 (ε 7900); (pH 7 buffer): λmax 271 (ε 12000), 278 (ε 8200); λmin 228 (ε 4700), 268 (ε 9300); (0.1N sodium hydroxide); λmax 270 (ε 10600); λmin 233 (ε 5400); λsh 257 (ε 9600): $^1$H nmr ($D_2O$) (HOD resonance was reduced by presaturation using the decoupler channel): δ 8.36 (broad s, 0.125 H, NH of "7 isomer"), 8.07 (broad s, 0.875 H, NH of "9 isomer"), 4.33 (t, 0.25 H, J = 7.0 Hz, $NCH_2$ of "7 isomer"), 4.08 (t, 1.75 H, J = 7.0 Hz, $NCO_2$ of "9 isomer"), 2.10 (dd, 2H, $J_{PH}$ = 16.6 and 9.2 Hz, $PCH_2P$), 1.95-1.60 (m, 4H, $PCH_2$ and $NCH_2C\underline{H}_2$), 1.60-1.20 (m, 6H, $PCH_2C\underline{H}_2C\underline{H}_2C\underline{H}_2$); $^{31}$P nmr ($H_2O$): δ 39.39 (s, 1P, phosphinyl), 15.98 (s, 1P, phosphonyl).

## Example 4

Preparation of (((7-(2-amino-1,6-dihydro-6-oxo-9H-purin-9-yl)heptyl)phosphinico)methyl)phosphonic acid (compound 4)

A) Preparation of diethyl 6-heptenylphosphonate

To a solution of diethyl methylphosphonate (13.99g, 92.0mmoles) in anhydrous tetrahydrofuran (20ml) (Aldrich Chem. Co.) at -60°C under a nitrogen atmosphere, was added a solution of n-butyl lithium in hexane (1.6M) (57.5ml, 92.0mmoles). The solution was stirred at -55°C for 20 minutes, and then a solution of 6-bromo-1-hexene (15g, 92.0mmoles) in anhydrous tetrahydrofuran (30ml) was added in a dropwise manner. The solution was allowed to slowly warm to ambient temperature as it stirred for 18 hours. The solution was concentrated to 60ml in vacuo, dissolved in water (100ml) and extracted with dichloromethane (4 x 300ml). The organic layer was spin evaporated in vacuo and the residual oil was purified by fractional distillation to give 12.77g (59.3%) of diethyl 6-heptenylphosphonate; bp - 155-168°C at 17 Torr: ms CI ($CH_4$): m/e = 235 ($MH^+$); $^1$H nmr ($CDCl_3$): δ 5.80-5.70 (m, 1H, $CH_2=C\underline{H}$), 5.00-4.87 (m, 2H, $C\underline{H}_2=CH$), 4.10-3.99 (m, 4H, $POCH_2$), 2.07-1.95 (m, 2H, $CH_2=CH\ CH_2$), 1.72-1.55 (m, 4H, $PCH_2$ and $CH_2=CHCH_2C\underline{H}_2$), 1.40-1.33 (m, 4H, $PCH_2C\underline{H}_2C\underline{H}_2$), 1.28 (t, 6H, J = 7.0 Hz, $POCH_2C\underline{H}_3$); $^{31}$P nmr (DMSO-$d_6$): δ 33.43 (s, phosphonyl).

B) Preparation of ethyl 6-heptenylphosphonochloridate

This compound was prepared in an analogous manner to that of Example 2 with replacement of diethyl 3-butenylphosphonate in Example 2A with diethyl 6-heptenylphosphonate. Evaporation of the dichloromethane under reduced pressure left 10.5g (92.4%) of ethyl 6-heptenylphosphonochloridate as clear oil: $^1$H nmr ($CDCl_3$): δ 5.80-4.89 (m, 1H, $CH_2=C\underline{H}$), 5.00-4.89 (m, 2H, $C\underline{H}_2=CH$), 4.35-4.15 (m, 2H, $POC\underline{H}_2$), 2.16-1.90 (m, 4H, $PC\underline{H}_2$ and $CH_2=CHC\underline{H}_2$), 1.77-1.64 (m, 2H, $CH_2=CHCH_2C\underline{H}_2$), 1.47-1.37 (m, 4H, $PC\underline{H}_2$ and $C\underline{H}_2C\underline{H}_2$), 1.36 (t, 3H, J = 7.1, $POCH_2C\underline{H}_3$).

C) Preparation of diethyl ((ethoxy(6-heptenyl)phosphinoyl)methyl)phosphonate

This compound was prepared in an analogous manner to that of Example 1 with the replacement of ethyl

4-pentenylphosphonochloridate in Example 1C with ethyl 6-heptenylphosphonochloridate. Fractional distillation gave 6.16g (41.9%) of diethyl ((ethoxy(6-heptenyl)phosphinoyl)methyl)phosphonate: bp 160-170°C at 0.2 Torr; ms 1C ($CH_4$): m/e = 341 ($MH^+$); $^1H$ nmr ($CDCl_3$): δ 5.82-5.72 (m, 1H, $CH_2$=C<u>H</u>), 5.0-4.89 (m, 2H, $C\underline{H}_2$=CH), 4.20-4.05 (m, 6H, $POC\underline{H}_2CH_3$), 2.37 (dd, 2H, $J_{PH}$ = 16.4 and 20.7 Hz), 2.10-2.00 (m, 2H, $CH_2$-$CHC\underline{H}_2$), 2.00-1.86 (m, 2H, $PCH_2$), 1.70-1.55 (m, 2H, $CH_2=CHCH_2C\underline{H}_2$), 1.45-1.3 (m, 2H, $PCH_2C\underline{H}_2C\underline{H}_2$), 1.33 (t, 6H, J = 7.0 Hz, $POCH_2C\underline{H}_3$), 1.32 (t, 3H, J = 7.0 Hz, $POCH_2C\underline{H}_3$): $^{31}P$ NMR (DMSO-$d_6$): δ 48.01 (d, 1P, $J_{pp}$ = 5.12 Hz, phosphinyl), 22.66 (d, 1P, J = 5.12 Hz, phosphonyl).

D) <u>Preparation of diethyl ((ethoxy(7-hydroxyheptyl)phosphinoyl)methyl)phosphonate</u>

A solution of diethyl ((ethoxy(6-heptenyl)phosphinoyl)methyl)phosphonate (2.0, 5.69mmoles) in anhydrous tetrahydrofuran (5ml) was added dropwise to a solution of borane (1.0M in tetrahydrofuran) (5.7ml, 5.7mmoles) in tetrahydrafuran (10ml) at -10°C under a nitrogen atmosphere. The solution was stirred for 2 hours while it warmed to ambient temperature. To this solution was added, dropwise, distilled water (6ml) followed by sodium perborate.4 $H_2O$ (2.62g, 17mmoles) (Aldrich Chemical Company) in portions. The mixture was stirred for 2 hours, and then spin evaporated <u>in</u> <u>vacuo</u> to dryness. Dichloromethane was added (3 x 150ml) during the evaporation to remove the last traces of water. The white residue was leached with ethyl acetate (3 x 150ml). The ethyl acetate solution was filtered and concentrated <u>in</u> <u>vacuo</u> to give 2.18g (100%) of diethyl ((ethoxy(7-hydroxyheptyl)phosphinoyl)methyl)phosphonate 0.75 hydrate as a thick oil: ms CI ($CH_4$): m/e = 359 ($MH^+$); $^1H$ nmr ($CDCl_3$): δ 4.22-4.05 (m, 6H, $POCH_2$), 3.32 (t, 2H, J = 6.4 Hz, $C\underline{H}_2OH$), 2.37 (dd, 2H, $J_{PH}$ a 16.5 and 20.8 Hz, $PCH_2P$), 2.05-1.80 (m, 3H, $PCH_2$ and OH), 1.80-1.50 (m, 4H, $PCH_2C\underline{H}_2$ and $HOCH_2C\underline{H}_2$). 1.48-1.30 (m, 6H, $PCH_2CH_2C\underline{H}_2C\underline{H}_2$-$C\underline{H}_2$), 1.33 (t, 6H, J = 7.0 Hz, $POCH_2CH_3$), 1.32 (t, 2H, J = 7.0 Hz, $POCH_2C\underline{H}_3$); $^{31}P$ nmr (DMSO-$d_6$): δ 47.79 (d, 1P, $J_{PP}$ = 5.2 Hz, phosphinyl), 22.39 (d, 1P, $J_{PP}$ = 5.2 Hz, phosphonyl).

E) <u>Preparation of diethyl ((ethoxy(7-((methylsulfonyl)oxy)heptyl) phosphinoyl)methyl)phosphonate</u>

This compound was prepared in an analogous manner to that of Example 1 with the replacement of diethyl ((ethoxy(5-hydroxypentyl)phosphinoyl)methyl)phosphonate in Example 1E with diethyl ((ethoxy(7-hydroxyheptyl)phosphinoyl)methyl)phosphonate. Evaporation of the chromatography solution gave 3.0g (80.5%) of diethyl ((ethoxy(7-((methylsulfonyl)oxy)heptyl)phosphinoyl)methyl)phosphonate monohydrate as a thick oil: ms CI($CH_4$): m/e = 437 ($MH^+$); $^1H$ nmr ($CDCl_3$): δ 4.25-4.00 (m, 8H, $SO_2OC\underline{H}_2$ + $POC\underline{H}_2$), 3.00 (s, 3H, $CH_3SO_3$), 2.37 (dd, 2H, $J_{PP}$ = 16.6 and 20.7 Hz, $PCH_2P$), 2.05-1.80 (m, 2H, $PC\underline{H}_2$), 1.80-1.50 (m, 6H, $C\underline{H}_2C\underline{H}_2CH_2CH_2C\underline{H}_2CH_2P$), 1.5-1.3 (m, 4H, $C\underline{H}_2C\underline{H}_2CH_2CH_2P$), 1.33 (t, 6H, J = 7.0 Hz, $POCH_2C\underline{H}_3$), 1,32 (t, 3H, J = 7.0 Hz, $POCH_2C\underline{H}_3$); $^{31}P$ nmr (DMSO-$d_6$): δ 47.78 (d, 1P, $J_{PP}$ = 5.2 Hz, phosphinyl), 22.39 (d, 1P, $J_{PP}$ = 5.2 Hz, phosphonyl).

F) <u>Preparation of diethyl (((7-(2-amino-6-(2-methoxyethoxy)-9H-purin-9-yl)heptyl)ethoxyphosphinoyl)methyl)phosphonate</u>

This compound was prepared in an analogous manner to that of Example 1 with the replacement of diethyl ((ethoxy(5-((methylsulfonyl)oxy)pentyl)phosphinoyl)methyl)phosphonate in Example 1F with diethyl ((ethoxy(7-((methylsulfonyl)oxy)heptyl)phosphinoyl)methyl)phosphonate. The chromatography solution was concentrated <u>in</u> <u>vacuo</u>, to give 1.43g (37.8%) of diethyl (((7-(2-amino-6-(2-methoxyethoxy)-9<u>H</u>-purin-9-yl)heptyl)ethoxyphosphinoyl)methyl)phosphonate as a thick oil: $^1H$ nmr (DMSO-$d_6$): δ 7.85 (s, 1H, purine 8-H), 6.36 (br s, 2H, $NH_2$), 4.50 (t, 2H, J = 4.7 Hz $OCH_2$), 4.20-3.82 (m, 8H, $POC\underline{H}_2$) and $NC\underline{H}_2$) 3.67 (t, 2H, J = 4.7 Hz, $C\underline{H}_2OCH_3$), 3.30 (s, 3H, $OCH_3$), 2.55 (dd, 2H, $J_{PH}$ = 16.5 and 19.2 Hz, $PCH_2P$), 1.90-1.60 (br, m, 2H, $NCH_2C\underline{H}_2$), 1.5-1.3 (br m, 2H, $PCH_2$), 1.30-1.00 (broad m, 17H, $PCH_2C\underline{H}_2C\underline{H}_2C\underline{H}_2CH_2$ and $POCH_2C\underline{H}_3$); $^{31}P$ (DMSO-$d_6$) δ 46.28 (d, 1P, $J_{PP}$ = 5.1 Hz, phosphinyl), 21.6 (d, 1P, $J_{PP}$ = 5.1 Hz, phosphonyl).

G) <u>Preparation of (((7-(2-amino-1.6-dihydro-6-oxo-9H-purin-9-yl)heptyl)phosphinico)methyl)phosphonic acid</u>

This compound was prepared in an analogous manner to that of Example 1 with the replacement at diethyl (((5-(2-amino-6-(2-methoxyethoxy)-9<u>H</u>-purin-9-yl)pentyl)ethoxyphosphinoyl)methyl)phosphonate in Example 1G with diethyl (((7-(2-amino-6-(2-methoxyethoxy)-9<u>H</u>-purin-9-yl)heptyl)ethoxyphosphinoyl)methyl)phosphoate. Lyophilization of the chromatography solution gave 0.497g (61.4%) of (((7-(2-amino-1,6-dihydro-6-oxo-9H-purin-9-yl)heptyl)phosphinico)methyl)phosphonic acid 1.3 ammonium salt 0.85 hydrate as white crystals: mp 140-145°C; ms $FAB^+$: m/e = 408 ($MH^+$); uv (0.1N hydrochloric acid): λmax 252 (ε 11700), 278 (ε 7800), λmin 267 (ε 7600); (pH 7 buffer): λmax 252 (12200), λsh 271 (ε 9300); (0.1N sodium hydroxide): λmax 268 (10600),

λsh 256 (9800); $^1H$ nmr (DMSO-$d_6$): δ 7.68 (s, 1H, purine H-8), 6.52 (broad s, 2H, $NH_2$), 5.00 (very broad s,$^+NH_4$ and $H_2O$), 3.91 (t, 2H, J = 7.1 Hz, $NCH_2$), 1.80-1.60 (broad m, 4H, $PCH_2P$ and $NCH_2C\underline{H_2}$), 1.50-1.35 (br, m, 4H, $PCH_2$ and $NCH_2CH_2$), 1.30-1.15 (br s, 6H, $PCH_2C\underline{H_2}C\underline{H_2}C\underline{H_2}$); $^{31}P$ nmr ($D_2O$): δ 43.13 (broad s, 1P, phosphinyl), 15.31 (broad s, 1P, phosphonyl).

Example 5

Preparation of ethyl hydrogen (((5-(2-amino-1,6-dihydro-6-oxo-9H-purin-9-yl)pentyl)phosphinico)methyl)phosphonate (compound 5)

A solution of diethyl (((5-(2-amino-6-(2-methoxyethoxy)-9H-purin-9-yl) pentyl)ethoxyphosphinoyl)methyl)phosphonate (0.400g, 0.78mmoles) in ethanol (5ml) and hydrochloric acid (IN) (10ml) was heated to reflux for 1 hour. The cooled reaction solution was spin evaporated in vacuo. The residue was dissolved in ethanol (10ml) containing potassium hydroxide 85% (1g). The solution was heated to reflux for 2 hours. The cooled solution was diluted with hydrochloric acid (1N) (20ml) and spin evaporated in vacuo. The residue was dissolved in water (400ml) and purified by anion exchange chromatography on DEAE-Sephadex A25 (-$HCO_3$ form) (21mm x 250mm column) with an aqueous ammonium bicarbonate buffer (0 to 1 molar gradient) (21). The appropriate fractions were combined and spin evaporated in vacuo. The residue was dissolved in water and lyophilized to give 0.174g (50%) of ethyl hydrogen (((5-(2-amino-1,6-dihydro-6-oxo-9H-purin-9-yl)pentyl)phosphinico)methyl)phosphonate as the monoammonium salt: mp 130-140°C; uv (0.1N hydrochloric acid): λmax 277 nm (ε 8300), 252 nm (ε 12400); λmin 269 nm (ε 8200); 227 nm (ε 3100); (pH 7 buffer): λmax 251 nm (ε 13,300); λmin 225 nm (ε 3400); λsh 270 nm (ε 10,000); (0.1N sodium hydroxide): λmax 268 nm (ε 11,400); λmin 231 nm (ε 4900); λsh 256 nm (ε 10,700); ms ($FAB^+$): m/e = 408 ($MH^+$); $^1H$ nmr (DMSO): δ 7.69 (s, 1H, H-8), 6.50 (broad s, 2H, $NH_2$), 4.8 (very broad s,$^+NH_4$ and $H_2O$), 3.91 (t, 2H, J = 7.1 Hz, $NCH_2$), 3.72 (dq, 2H, $J_{PH}$ = 7.1 Hz, $J_{HH}$ - 7.1 Hz, $OCH_2$), 1.76 (dd, 2H, $J_{PH}$ = 15.4 Hz, 17.4, $PCH_2P$), 1.75-1.6 (m, 2H, $CH_2$), 1.60-1.40 (m, 4H, $CH_2$-and $CH_2$), 1.35-1.2 (m, 2H, $CH_2$), 1.10 (t, 3H, J = 7.0 Hz, $CH_3$).

Example 6

Preparation of ((((3-(2-amino-1,6-dihydro-6-oxo-9H-purine-9-yl)propoxy)methyl)phosphinico)methyl)phosphonic acid (compound 6)

A) Preparation of diisopropyl 2-propenyloxymethyl phosphonate

Triisopropyl phosphite (82.25g, 0.395moles) (Aldrich Chem. Co.) was added to 1-chloromethoxy-2-propene (42.11g, 0.395moles) (J. Colonge and P. Boisole, Bull. Soc. Chim., 1956, 824) at 90°C dropwise over 30 minutes. The solution was refluxed with an oil bath for 2 hours while the bath temperature was raised to 135-140°C. The reaction reflux condensor was then exchanged for a distillation head and isopropyl chloride was collected. When the internal reaction temperature reached 135°C, the heat was removed and the reaction was allowed to cool. Fractional distillation then gave 13.8g (15.9%) of diisopropyl 2-propenyloxymethylphosphonate as a clear oil: bp 133-135°C at 15 Torr; $^1H$ nmr ($CDCl_3$): δ 5.95-5.80 (m, 1H, $CH_2$=C$\underline{H}$), 5.34-5.21 (m, 2H, $C\underline{H_2}$=CH), 4.82-4.70 (m, 2H, POC$\underline{H}$), 4.11 (d, 2H, J = 5.7 Hz, $CH_2$=$CHCH_2$), 3.71 (d, 2H, $J_{PH}$ = 8.7 Hz, $PCH_2O$), 1.34 (m, 12H, $POCH(C\underline{H_3})_2$).

B) Preparation of isopropyl 2-propenyloxymethylphosphonochloridate

The compound was prepared in an analogous manner to that of Example 2 with the replacement of diethyl 3-butenylphosphonate in Example 2A with diisopropyl 2-propenyloxyphosphonate. The volatiles were removed by spin evaporation in vacuo at 60°C with the addition of dichloromethane (6 x 250ml) to give 17.73g (98.5%) of isopropyl 2-propenyloxymethylphosphonochloridate.

C) Preparation of-diisopropyl ((isopropoxy(2-propenyloxymethyl)phosphinoyl)methyl)phosphonate

This compound was prepared in an analogous manner to that of Example 1 with the replacement of ethyl 4-pentenylphosphonochloridate and diethyl methylphosphonate in Example 1C with isopropyl 2-propenyloxymethylphosphonochloridate and diisopropyl methyl-phosphonate (Alfa Catalog Chemicals, Morton Thiakal,Inc.). Fractional distillation gave 14.5g (48.7%) of diisopropyl ((isopropoxy(2-propenyloxymethyl)phosphinoyl)methyl)phosphonate: bp 120-144°C at 0.15 Torr; $^1H$ nmr ($CDCl_3$): δ 5.95-5.80 (m, 1H, $CH_2$=C$\underline{H}$), 5.35-

5.20 (m, 2H, C$\underline{H}_2$-CH), 4.86-4.70 (m, 3H, POCH), 4.15-4.08 (m, 2H, $CH_2$=CHC$\underline{H}_2$), 3.95-3.78 (m, 2H, $PCH_2O$), 2.58-2.31 (m, 2H, $PCH_2P$), 1.40-1.28 (m, 18H, POCH(C$\underline{H}_3)_2$).

D) Preparation of diisopropyl ((isopropoxy(3-hydroxy-1-propoxymethyl)phosphinoyl)methyl)phosphonate

This compound was prepared in an analogous manner to that of Example 1 with the replacement of diethyl ((ethoxy(4-pentenyl)phosphinoyl)methyl)phosphonate in Example 1D with diisopropyl ((isopropoxy(2-propenyloxomethyl)phosphinoyl)methyl)phosphonate. The ethyl acetate extraction solutions were concentrated in vacuo to give 3.0g (94.6%) of diisopropyl ((isopropoxy(3-hydroxy-1-propoxymethyl)phosphinoyl)methyl)phosphonate as a pale yellow oil: ms CI ($CH_4$): m/e = 375 ($MH^+$); $^1$H nmr ($CDCl_3$): δ 4.90-4.65 (m, 3H, POC$\underline{H}$($CH_3)_2$, 3.91 (d, 2H, $J_{PH}$ = 6.9 Hz, PC$\underline{H}_2$), 3.80-3.68 (m, 4H, OC$\underline{H}_2$$CH_2$C$\underline{H}_2$OH), 2.44 (dd, 1H, $J_{PH}$ = 18.0 and 20.5 Hz, PC$\underline{H}$HP), 2.42 (dd, 1H, $J_{PH}$ = 18.5 and 21.0 Hz, PCH$\underline{H}$P), 1.85-1.60 (m, 3H, $CH_2$C$\underline{H}$$CH_2$ and OH), 1.40-1.25 (m, 18H, POCH(C$\underline{H}_3)_2$) $^{31}$P ($CDCl_3$): δ 40.25 (s, 1P, phosphinyl), 18.21 (s, 1P, phosphonyl).

E) Preparation of diisopropyl ((isopropoxy(3-((methylsulfonyl)oxy)propoxy)methyl)phosphinoyl)methyl)phosphonate

This compound was prepared in an analogous manner to that of Example 1 with the replacement of diethyl ((ethoxy(5-hydroxypentyl)phosphinoyl)methyl)phosphonate in Example 1E with diisopropyl ((isopropoxy(3-hydroxy-1-propoxymethyl)phosphinoyl)methyl)phosphonate. The chromatography solutions were concentrated in vacuo to give 3.48, (96.9%) of diisopropyl ((isopropoxy(3-((methylsulfonyl)oxy)propoxy)methyl) phosphinoyl)methyl)phosphonate as an oil: $^1$H nmr ($CDCl_3$): δ 4.85-4.70 (m, 3H, POCH($CH_3)_2$), 4.35 (t, 2H, J = 6.2 Hz, $SOCH_2$), 3.95-3.85 (m, 2H, $PCH_2P$), 2.15-2.00 (m, 2H, $CH_2$C$\underline{H}_2$$CH_2$), 1.45-1.30 (m, 18H, POCH(C$\underline{H}_3)_2$).

F) Preparation of diisopropyl (((3-(2-amino-6-(2-methoxyethoxy)-9H-purin-9-yl)propoxymethyl)ethoxyphosphinoyl)methyl)phosphonate

This compound was prepared in an analogous manner to Example 1 with the replacement of diethyl ((ethoxy(5-((methylsulfonyl)oxy)pentyl)phosphinoyl)methyl)phosphonate in Example 1F with diisopropyl ((isopropoxy(3-((methylsulfonyl)oxy)propoxy)methyl)phosphinoyl)methyl)phosphonate. The chromatography solution was concentrated in vacuo to give 1.128g (26.1%) of diisopropyl (((3(2-amino-6-(2-methoxyethoxy)-9H-purin-9-yl)propoxymethyl)ethoxyphosphinoyl)methyl)phosphonate as a pale yellow syrup: $^1$H nmr (DMSO-$d_6$): δ 7.86 (s, 1H, purine H-8), 6.39 (br S, 2H, $NH_2$), 4.75-4.53 (m, 3H, POC$\underline{H}$-($CH_3)_2$), 4.49 (t, 2H, J = 6.0 Hz, OC$\underline{H}_2$), 4.06 (t, J = 6.8 Hz, $NCH_2$) 3.79 (d, 2H, J = 6.6 Hz, $PCH_2O$), 3.67 (t, 2H, J = 4.6 Hz, C$\underline{H}_2$O) 3.55-3.41 (m, 2H, C$\underline{H}_2$$OCH_2P$), 3.29 (S, 3H, $SCH_3$), 2.55 (dd, 2H, $J_{PH}$=17.1 and 20.7 Hz, $PCH_2P$), 2.05-1.90 (m, 2H, $CH_2$C$\underline{H}_2$$CH_2$), 1.23 (d, 18H, J = 6.1 Hz, POCH($CH_3)_2$); $^{31}$P nmr (DMSO-$d_6$): δ 40.05 (d, 1P, J = 5.3 Hz, phosphinyl), 19.42 (d, 1P, J = 5.3 Hz, phosphonyl).

G) Preparation of (((13-(2-amino-1.6-dihydro-6-oxo-9H-purine-9-yl)propoxy)methyl)phosphinico)methyl)phosphonic acid

This compound was prepared in an anlogous manner to that of Example 1 with the replacement of diethyl (((5-(2-amino-6-(2-methoxyethoxy)-9H-purin-9-yl)pentyl)ethoxyphosphinoyl)methyl)phosphonate in Example 1G with diisopropyl (((3-(2-amino-6-(2-methoxyethoxy)-9H-purin-9-yl)propoxymethyl)ethoxyphosphinoyl)methyl)phosphonate. Lyophilization of the chromatography solution gave 0.542g (80%) of (((3-(2-amino-1,6-dihydro-6-oxo-9H-purine-9-yl)propoxy)methyl)phosphinico)methyl)phosphonic acid as the 1.2 ammonium salt 0.5 hydrate: mp - 153-163°C; ms ($FAB^+$): m/e = 382 ($MH^+$); uv (O.1N hydrochloric acid): λmax 276.5 (ε 7800); 252 (ε 11700); λmin 270 (ε 7600), 226 (ε 4000); (pH 7 buffer): 251 (ε 12400), λmin 225 (ε 4300), λsh 269.5 (ε 9200); (0.1N sodium hydroxide): λmax 267.5 (ε 12700), 255.5 (ε 12100); λmin 232.5 (ε 8000); $^1$H nmr ($D_2O$)): δ 8.27 (broad s, 1H, purine H-8), 4.23 (t, 2H, J = 6.9 Hz, $NCH_2$), 3.64 (d, 2H, $J_{PH}$ = 7.3 Hz, PC$\underline{H}_2$O), 3.56 (t, 2H, J = 5.9 Hz, $CH_2O$), 2.20-2.04 (m, 4H, $PCH_2P$ and $CH_2$C$\underline{H}_2$$CH_2$); $^{31}$P nmr ($D_2O$): δ 31.54 (s, 1P, phosphinyl, 16.09 (s, 1P, phosphonyl).

Enzymology-Purine nucleoside phosphorylase (PNPase) was purified from human erythrocytes by the procedure of Agarwal et al (1). PNPase and xanthine oxidase were desalted before use (2). The desalted enzyme preparations were stable for at least six months when stored at -70°C in 10 mM Tris=HCl buffer, pH 7.5. PNPase was assayed spectrophotometrically as previously described (2). In addition to PNPase, the reaction mixtures contained 0.1 mM inosine, 1 mM potassium phosphate, 100 mM Tris=HCl buffer (pH 7.5), 2μM $ZnCl_2$, and 0.2

U/ml of xanthine oxidase. Phosphorolysis of inosine was measured at 293 nm ($\Delta\Sigma$ = 12.5 $mM^{-1}\cdot cm^{-1}$). The percent inhibition (%I) of PNP by a single concentration of inhibitor was measured (3). Sufficient inhibitor was added to the reaction in order to give a percent inhibition of approximately 50% whenever possible. The apparent inhibition constant ($K_{i,}$)was calculated from the percent inhibition assuming competitive inhibition (4), as has been observed for acyclovir and its analogues and their phosphorylated derivatives (2).

1.Agarwal, R.P., Agarwal, K.C., and Parks, R.E., Jr. (1978) (Methods Enzymol. 51, 581-586.

2.Tuttle, J.V., and Krenitsky, T.A. (1984) J.Biol.Chem 259, 4065-4069.

3. $$\%I - 100\,[I]\,\frac{(100/\%I)-1}{1+([S]/K_{m'})}$$ ; where [I] - inhibitor concentration, [S] = inosine concentration = 0.1 mM, and $K_{m'}$ = apparent $K_m$ for inosine = 0.04 mM.

Inhibition of Purine Nucleoside Phosphorylase

| | Ki (μM) |
|---|---|
| Compound 1 | .0026 |
| Compound 2 | .077 |
| Compound 5 | 0.87 |
| Compound 6 | 0.0045 |
| Compound 3 | .0031 |
| Compound 4 | .0083 |

The following examples illustrate pharmaceutical formulations according to the present invention:-

Injectable solution

A solution for intramuscular injection may be prepared by mixing:-

| | | |
|---|---|---|
| Compound of formula (I) | 9.5 | parts by weight |
| Dimethyl sulphoxide | 19.0 | parts by weight |
| Sorbitan monooleate | 4.5 | parts by weight |
| Corn oil | 67.0 | parts by weight |
| | 100.0 | |

Injectable solution

| | | |
|---|---|---|
| Compound of formula (I) | 5 | parts by weight |
| N-methyl-pyrollidone | 48.3 | parts by weight |
| Tween 80 | 2 | parts by weight |
| Span 80 | 4.7 | parts by weight |
| Miglyol 812 | 40 | parts by weight |
| | 100.0 | |

Tablet

| | |
|---|---|
| Compound of formula (I) | 25.0 mg |
| Lactose BP | 48.5 mg |
| Microcrystalline Cellulose BP ("Avicel pH 101") | 10.0 mg |
| Low-substituted Hydroxypropyl; Cellulose BP ("LHPC LH-11") | 10.0 mg |
| Sodium Starch Glycollate BP ("Explotab") | 3.0 mg |
| Povidone BP ("K30") | 3.0 mg |
| Magnesium Stearate BP | 0.5 mg |
| | 100.0 mg |

Oral suspension

| | |
|---|---|
| Compound of formula (I) | 50 mg |
| Avicel RC 591 | 75 mg |
| Sucrose syrup | 3.5 ml |
| Methylhydroxybenzoate | 5 mg |
| Colour | 0.01% w/v |
| Cherry flavour | 0.1 % v/v |
| Tween 80 | 0.2 % v/v |
| Water | to 5 ml |

Injectable suspension

| | |
|---|---|
| Compound of formula (I) | 100 mg |
| Polyvinyl pyrrolidone (PVP) | 170 mg |
| Tween 80 | 0.2% v/v |
| Methylhydroxybenzoate | 0.1% w/v |
| Water for Injection | to 3 ml |

Capsule

| | |
|---|---|
| Compound of formula (I) | 100 mg |
| Starch 1500 | 150 mg |
| Magnesium stearate | 2.5 mg |
| filled into a hard gelatin capsule | |

Suspension for Nebulisation

| | | |
|---|---|---|
| Compound of formula (I), sterile | | 1.0 mg |
| Water for Injections | to | 10.0 ml |

Disperse the compound of formula (I) in the Water for Injections previously sterilised in a sterile container. Fill in to sterile glass ampoules, 10 ml/ampoule under aseptic conditions, and seal each ampoule by fusion of the glass.

Aerosol Formulation

| | | |
|---|---|---|
| Compound of formula (I), micronised | | 1.0 mg |
| Aerosol propellant | to | 5.0 ml |

Suspend the micronised compound of formula (I) in the aerosol propellant. Fill this suspension into preformed aerosol cannisters, 5 ml/cannister under pressure, through the valve orifice.

Powder Inhalation

| | | |
|---|---|---|
| Compound of formula (I), micronised | | 1.0 mg |
| Lactose | | 29.0 mg |

Triturate and blend the micronised compound of formula (I) with the lactose. Fill the resulting powder blend into hard gelatin capsule shells, 30 mg per capsule.

Nasal Drops

| | | |
|---|---|---|
| Compound of formula (I) | | 100.0 mg |
| Methylhydroxybenzoate | | 10.0 mg |
| Water for Injections | to | 10.0 ml |

Disperse the compound of formula (I) and the methylhydroxybenzoate in the Water for Injections. Fill this suspension into suitable dropper bottles, 10 ml/bottle, and close by securing the dropper nozzle and bottle cap.

**Claims**

**1)** A compound of the formula (I):

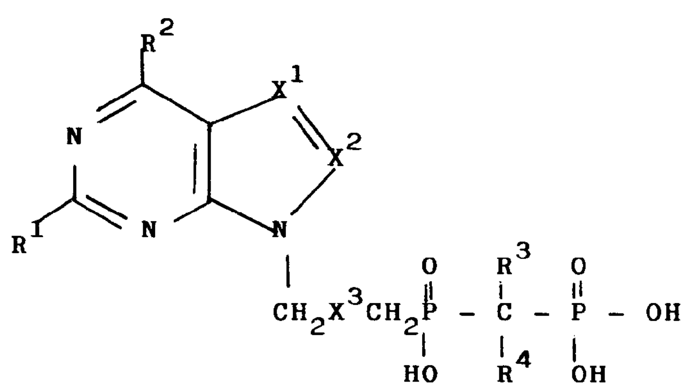

(I)

or a pharmaceutically acceptable salt or ester thereof, wherein $X^1$ is nitrogen or a group CH and $X^2$ is nitrogen or a group $CR^5$ wherein $R^5$ is hydrogen, hydroxy, halo or a group $NHR^6$ wherein $R^6$ is hydrogen or a $C_{1-4}$ alkyl or $C_{1-4}$ alkylcarbonyl group, $R^1$ is amino or hydrogen; $R^2$ is hydrogen, halo, SH, a group $NHR^7$ wherein $R^7$ is hydrogen or a $C_{1-4}$ alkyl or $C_{1-4}$ alkylcarbonyl group or $R^2$ is a group $OR^8$ wherein $R^8$ is hydrogen, $C_{1-4}$ alkyl or benzyl, $R^3$ and $R^4$ are the same or different and each is hydrogen or halo, $X^3$ is a chain containing m $CH_2$ groups and n oxygen atoms, m being 1, 2, 3, 4, or 5 and n being 0, 1 or 2 the sum of m and n being 2, 3, 4, 5, or 6.

**2)** A compound according to claim 1 where $X^1$ is nitrogen and $X^2$ is nitrogen, or a group $CR^5$ wherein $R^5$ is hydrogen, halo or $NH_2$.

**3)** A compound according to either claim 1 or 2 wherein $R^1$ is hydrogen, or $NH_2$.

**4)** A compound according to any one of claims 1 to 3 wherein $R^3$ and $R^4$ are each hydrogen or fluoro, n is 0 or 1, and the sum of m and n is 2, 3, 4 or 5.

**5)** A compound according to any one of claims 1 to 4 wherein $X^3$ is a group $(CH_2)_3$, $(CH_2)_4$ or $(CH_2)_2O$.

**6)** A compound selected from

(((4-(2-amino-1,6-dihydro-6-oxo-9H-purin-9-yl)butyl)phosphinico)methyl)phosphonic acid

(((5-(2-amino-1,6-dihydro-6-oxo-9H-purin-9-yl)pentyl)phosphinico) methyl)phosphonic acid

(((6-(2-amino-1,6-dihydro-6-oxo-9H-purin-9-yl)hexyl)phosphinico)methyl)phosphonic acid

(((7-(2-amino-1,6-dihydro-6-oxo-9H-purin-9-yl)heptyl)phosphonico) methyl)phosphonic acid

(((3-(amino-1,6-dihydro-6-oxo-9H-purine-9-yl)propoxy)methyl)phosphinico)methyl)phosphonic acid

or a pharmaceutically acceptable salt or ester thereof.

**7)** A process for the preparation of a compound of formula I, or a pharmaceutically acceptable salt or ester thereof, as defined according to any one of claims 1 to 6 comprising:

a) the reaction of a compound

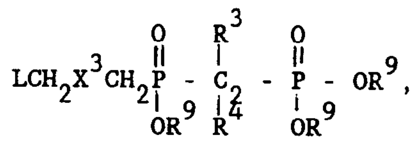

wherein $R^9$ is a $C_{1-6}$ alkyl or $C_{7-8}$ aralkyl protecting group and L is a leaving group, with a compound of the formula (II):

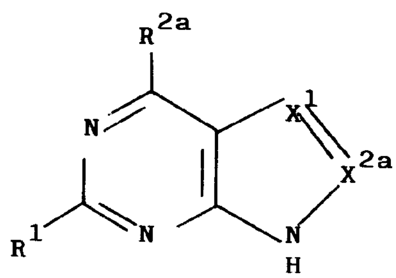

(II)

wherein $R^1$ and $X^1$ are as hereinbefore defined and $R^{2a}$ and $X^{2a}$ are groups $R^2$ and $X^2$ as hereinbefore defined or such groups substituted by a suitable protecting group, and thereafter optionally removing any protecting groups present

b) when $X^3$ contains one or two oxygen atoms, the reaction of a compound

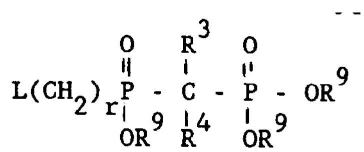

wherein L $R^3$, $R^4$ and $R^9$ are as hereinbefore defined, with a compound of the formula (III):

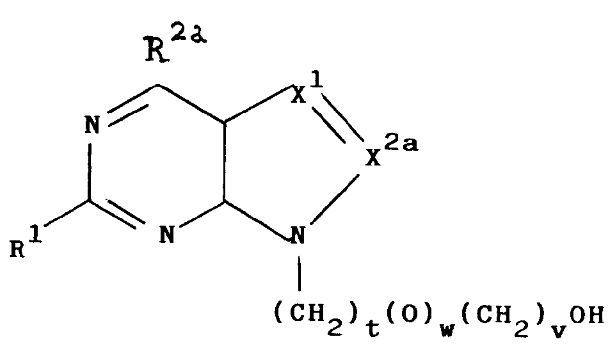

(III)

wherein $R^1$, $R^{2a}$, $X^1$ and $X^{2a}$ are as hereinbefore defined and t is 1, 2, 3, or 4, w is 0 or 1 and v is 0, 1, 2 or

3, the sum of r, t, w and v being 5, 6 or 7.

c) the conversion of one compound of the formula (I) from another compound of the formula (I).

**8)** A pharmaceutical formulation which comprises a compound of the formula (I) or a pharmaceutically acceptable salt or ester thereof as defined according to any one of claims 1 to 6 and a pharmaceutically acceptable carrier.

**9)** A compound of the formula (I) or a pharmaceutically acceptable salt or ester thereof as defined according to any one of claims 1 to 6 for use in medicine.

**10)** Use of a compound of the formula (I) or a pharmaceutically acceptable salt or ester thereof as defined according to any one of claims 1 to 6 in the manufacture of a medicament for treatment of antoimmune diseases.

**Claims for the following Contracting States : Es, Gr**

**1)** A process for the preparation of a compound of the formula (I):

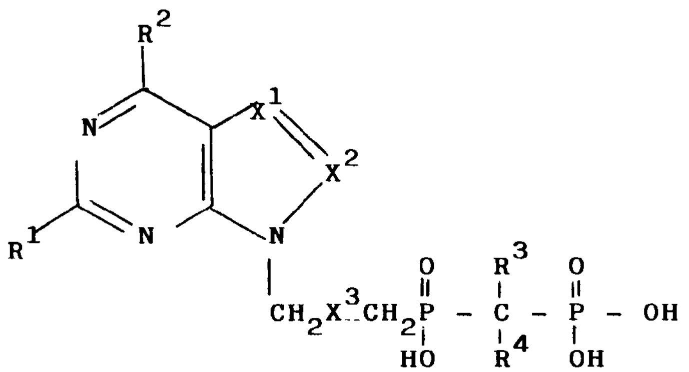

(I)

or a pharmaceutically acceptable salt or ester thereof, wherein $X^1$ is nitrogen or a group CH and $X^2$ is nitrogen or a group $CR^5$ wherein $R^5$ is hydrogen, hydroxy, halo or a group $NHR^6$ wherein $R^6$ is hydrogen or a $C_{1-4}$ alkyl or $C_{1-4}$ alkylcarbonyl group, $R^1$ is amino or hydrogen; $R^2$ is hydrogen, halo, SH, a group $NHR^7$ wherein $R^7$ is hydrogen or a $C_{1-4}$ alkyl or $C_{1-4}$ alkylcarbonyl group or $R^2$ is a group $OR^8$ wherein $R^8$ is hydrogen, $C_{1-4}$ alkyl or benzyl, $R^3$ and $R^4$ are the same or different and each is hydrogen or halo, $X^3$ is a chain containing m $CH_2$ groups and n oxygen atoms, m being 1, 2, 3, 4, or 5 and n being 0, 1 or 2 the sum of m and n being 2, 3, 4, 5, or 6; which comprises:

a) the reaction of a compound

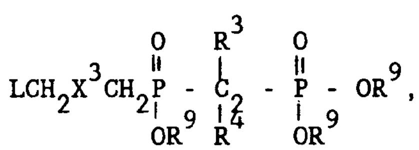

wherein $R^9$ is a $C_{1-6}$ alkyl or $C_{7-8}$ aralkyl protecting group and L is a leaving group, with a compound of the formula (II):

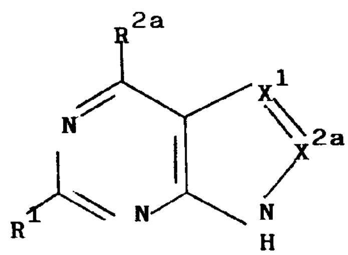

(II)

wherein $R^1$ and $X^1$ are as hereinbefore defined and $R^{2a}$ and $X^{2a}$ are groups $R^2$ and $X^2$ as hereinbefore

defined or such groups substituted by a suitable protecting group, and thereafter optionally removing any protecting groups present

b) when $X^3$ contains one or two oxygen atoms, the reaction of a compound

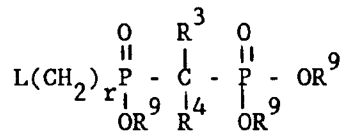

wherein L $R^3$, $R^4$ and $R^9$ are as hereinbefore defined, with a compound of the formula (III):

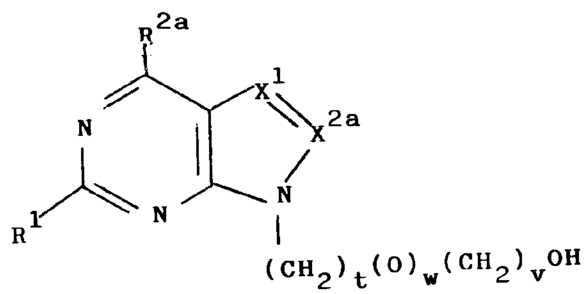

(III)

wherein $R^1$, $R^{2a}$, $X^1$ and $X^{2a}$ are as hereinbefore defined and t is 1, 2, 3, or 4, w is 0 or 1 and v is 0, 1, 2 or 3, the sum of r, t, w and v being 5, 6 or 7.

c) the conversion of one compound of the formula (I) from another compound of the formula (I).

**2)** A process for the preparation of a compound according to claim 1 where $X^1$ is nitrogen and $X^2$ is nitrogen, or a group $CR^5$ wherein $R^5$ is hydrogen, halo or $NH_2$.

**3)** A process for the preparation of a compound according to either claim 1 or 2 wherein $R^1$ is hydrogen, or $NH_2$.

**4)** A process for the preparation of a compound according to any one of claims 1 to 3 wherein $R^3$ and $R^4$ are each hydrogen or fluoro, n is 0 or 1, and the sum of m and n is 2, 3, 4 or 5.

**5)** A process for the preparation of a compound according to any one of claims 1 to 4 wherein $X^3$ is a group $(CH_2)_3$, $(CH_2)_4$ or $(CH_2)_2O$.

**6)** A process for the preparation of a compound according to any one of claims 1 to 5 selected from

(((4-(2-amino-1,6-dihydro-6-oxo-9H-purin-9-yl)butyl)phosphinico)methyl)phosphonic acid

(((5-(2-amino-1,6-dihydro-6-oxo-9H-purin-9-yl)pentyl)phosphinico) methyl)phosphonic acid

(((6-(2-amino-1,6-dihydro-6-oxo-9H-purin-9-yl)hexyl)phosphinico)methyl)phosphonic acid

(((7-(2-amino-1,6-dihydro-6-oxo-9H-purin-9-yl)heptyl)phosphonico) methyl)phosphonic acid

(((3-(amino-1,6-dihydro-6-oxo-9H-purine-9-yl)propoxy)methyl)phosphinico)methyl)phosphonic acid

or a pharmaceutically acceptable salt or ester thereof.

**7)** A pharmaceutical formulation which comprises a compound of the formula (I) or a pharmaceutically acceptable salt or ester thereof as defined according to any one of claims 1 to 6 and a pharmaceutically acceptable carrier.

**8)** A compound of the formula (I) or a pharmaceutically acceptable salt or ester thereof as defined according to any one of claims 1 to 6 for use in medicine.

**9)** Use of a compound of the formula (I) or a pharmaceutically acceptable salt or ester thereof as defined according to any one of claims 1 to 6 in the manufacture of a medicament for treatment of antoimmune diseases.

European Patent Office

# EUROPEAN SEARCH REPORT

Application Number

EP 91 30 8716

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 161 955 (MERCK & CO.)<br>* Whole document *<br>--- | 1 | C 07 F 9/6561<br>A 61 K 31/675 |
| A | EP-A-0 343 133 (MEDIVIR AKTIEBOLAG)<br>* Whole document *<br>----- | 1 | |
| | | | TECHNICAL FIELDS SEARCHED (Int. Cl.5) |
| | | | C 07 F 9/00<br>A 61 K 31/00 |

The present search report has been drawn up for all claims

| Place of search | Date of completion of the search | Examiner |
|---|---|---|
| THE HAGUE | 19-12-1991 | OUSSET J-B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)